# EUROPEAN PATENT SPECIFICATION

(11) **EP 3 928 700 B1**
(45) Date of publication and mention of the grant of the patent: **31.07.2024**
(21) Application number: 20792106.5
(22) Date of filing: 10.04.2020
(51) Int. Cl.: A61B 5/16, A61B 5/024

(54) **STRESS EVALUATION CALIBRATION METHOD, DEVICE AND STORAGE MEDIUM**
VERFAHREN, VORRICHTUNG UND SPEICHERMEDIUM ZUR KALIBRIERUNG DER STRESSBEURTEILUNG
PROCÉDÉ, DISPOSITIF DE CALIBRAGE D'ÉVALUATION DU STRESS ET SUPPORT DE MÉMOIRE

(30) Priority: 16.04.2019 CN 201910304393
(43) Date of publication of application: 29.12.2021
(73) Proprietor: Huawei Technologies Co., Ltd., Shenzhen, Guangdong 518129 (CN)
(72) Inventor: FU, Xiaoyu, Shenzhen, Guangdong 518129 (CN); XU, Peida, Shenzhen, Guangdong 518129 (CN)
(74) Representative: Isarpatent
(86) International application number: PCT/CN2020/084229
(87) International publication number: WO 2020/211702

(56) References cited:
- WO-A1-2019/037045
- CN-A- 104 490 407
- CN-A- 107 430 640
- CN-A- 108 601 566
- CN-A- 109 276 241
- CN-A- 109 276 241
- CN-A- 110 192 872
- US-A1- 2010 331 724
- US-A1- 2012 277 603
- US-A1- 2015 120 205
- US-A1- 2015 305 675
- US-A1- 2018 078 189

## Description

Priority is claimed to Chinese Patent Application No. 201910304393.8, filed with the China National Intellectual Property Administration on April 16, 2019 and entitled "STRESS EVALUATION AND CALIBRATION METHOD AND APPARATUS, AND STORAGE MEDIUM".

### TECHNICAL FIELD

This disclosure generally relates to the field of information processing technologies, and the invention in particular relates to a stress evaluation and calibration method and apparatus, and a storage medium.

### BACKGROUND

Psychological stress is a physiological change and a mood swing of a person that are caused by a change in an external environment and an internal state of an organism, usually accompanied by a positive or negative mood. Qualitative and quantitative assessment of psychological stress of a user is not only helpful to assist in an early warning of a physical condition, but also can assist the user in properly arranging a work plan, thereby improving work efficiency. Therefore, how to accurately evaluate people's psychological stress gradually becomes one of important issues concerned and researched in the industry.

In the conventional technology, with rise and portability of wearable devices, the wearable devices gradually become new carriers for user stress. A stress evaluation result may be obtained based on a psychological parameter signal detected by the wearable device and an existing assessment system. Then, the stress evaluation result is calibrated based on user self-evaluation information obtained through sampling before or after evaluation. Finally, an actual stress assessment result is obtained.

However, in the foregoing solution for calibrating the stress evaluation result, the user self-evaluation information needs to be obtained in a manner that the user answers a question. Due to subjective uncertainty of the user, problems of low accuracy of the evaluation result and poor user experience exist.

US 2010/331 724 A1 relates to a determination of a characteristic blood pressure. The characteristic blood pressure is derived from a patient's blood pressure in a suitable manner. For example, the characteristic blood pressure is obtained by analyzing the patient's blood pressure following a most recent triggering event, such as the patient's interaction with a clinician. An initial blood pressure reading may be made using a calibration device (e.g., a blood pressure cuff) such that at a time zero, the pressure value for the patient is equivalent to an initial blood pressure reading. A blood pressure curve also may be used to derive information about patient anxiety, which may be useful during a clinician exam or in situations where the patient's truthfulness is being assessed (e.g., during a lie detector test). For example, a difference between a patient's initial blood pressure reading and a characteristic or baseline blood pressure may be an indicator of patient stress by quantifying the change in the patient's blood pressure as a possible result of the triggering event. A stress indicator or relaxation time can be normalized to remove some statistical error before being used in patient assessment. For example, a stress indicator may be normalized by an initial blood pressure, a characteristic blood pressure, or a weighted mean of the two blood pressure readings. A relaxation time can be normalized by an average value of the relaxation time observed in a representative patient population.

US 2015/305 675 A1 relates to a wearable stress-testing device. A method begins by collecting, by a sensor of a wearable stress-testing device, data or measurements. The method also includes comparing, by a processing device coupled to the wearable stress-testing device, the data or the measurements collected with crowdsourced data, i.e. a measurement or data set or a measurement or measurement or data set collected from a group of individuals, to determine a measurement range for a medical condition. The crowdsourced data may be stored on the wearable stress-testing device, may be stored on a server or cloud-based storage, and/or may be stored in another location.. The crowdsourced data can be categorized based on criteria, such as age, gender, weight, ethnicity, race, fitness level, geographical information, environmental, or other demographic criteria. In this manner, a characteristic of the user of the wearable stress-testing device can be mapped to individuals in the group with similar characteristics.

### SUMMARY

The object of the present invention is to provide a stress evaluation and calibration method and apparatus, and a storage medium, to resolve problems of low accuracy of an existing stress evaluation result and poor user experience. This object is solved by the attached independent claims and further embodiments and improvements of the invention are listed in the attached dependent claims. Hereinafter, up to the "brief description of the drawings", expressions like "...aspect according to the invention", "according to the invention", or "the present invention", relate to technical teaching of the broadest embodiment as claimed with the independent claims. Expressions like "implementation", "design", "optionally", "preferably", "scenario", "aspect" or similar relate to further embodiments as claimed, and expressions like "example", "...aspect according to an example", "the disclosure describes", or "the disclosure" describe technical teaching which relates to the understanding of the invention or its embodiments, which, however, is not claimed as such and designated here as non-claimed.

A first aspect according to the present invention provides a stress evaluation and calibration method, applicable tc an electronic device or a server. The method includes:
obtaining a physiological parameter signal of a user;
determining a smallest stress state value of the user within preset duration based on an eigenvalue vector of the physiological parameter signal and a stress evaluation system;
determining calibration information based on a smallest reference stress value of a group to which the user belongs and the smallest stress state value; and
calibrating, by using the calibration information, a stress state value output by the stress evaluation system, to determine a theoretical stress state value of the user.

According to the invention, the calibration information of the stress evaluation system is determined based on the eigenvalue vector of the obtained physiological parameter signal of the user and the smallest reference stress value of the group to which the user belongs. The stress state value output by the stress evaluation system is calibrated by using the calibration information, to determine the theoretical stress state value of the user. In other words, in this technical solution, the user does not need to provide a self-evaluation comment, and the calibration information may be automatically generated. In this way, a stress evaluation result is automatically calibrated, to improve evaluation accuracy, and active participation of the user is not required, to improve user experience.

According to an embodiment of the invention of the first aspect, the determining a smallest stress state value of the user within preset duration based on an eigenvalue vector of the physiological parameter signal and a stress evaluation system includes:
obtaining an eigenvalue vector corresponding to the physiological parameter signal at each moment within the preset duration;
for the eigenvalue vector at each moment, inputting the eigenvalue vector into the stress evaluation system, to obtain a stress state value of the user at each moment; and
determining the smallest stress state value of the user within the preset duration based on the stress state value of the user at each moment within the preset duration.

According to the invention, the smallest stress state value of the user within the preset duration, namely, a stress state value at a most relaxed moment, may be determined based on the eigenvalue vector of the physiological parameter signal. This stress state value provides an implementation possibility for subsequently determining the calibration information.

According to the invention, the eigenvalue vector includes a plurality of eigenvalue components, and the stress state value of the user at each moment is obtained by performing weighted summation based on each eigenvalue component in the eigenvalue vector and a weight value corresponding to each eigenvalue component.

In another possible implementation of the first aspect, before the determining calibration information based on a smallest reference stress value of a group to which the user belongs and the smallest stress state value, the method further includes:
obtaining basic information of the user;
determining a group identifier of the user based on the basic information; and
querying a stress value database based on the group identifier of the user, to determine the smallest reference stress value of the group to which the user belongs, where the stress value database stores a correspondence between a group identifier and a reference stress range.

In this embodiment, the smallest reference stress value of the group to which the user belongs may be determined based on the basic information of the user. In this way, the calibration information of the stress evaluation system may be automatically determined without the active participation of the user, to improve user experience.

In still another possible implementation of the first aspect, the calibrating, by using the calibration information, a stress state value output by the stress evaluation system, to determine a theoretical stress state value of the user includes:
inputting the eigenvalue vector of the physiological parameter signal into the stress evaluation system to obtain a predicted stress state value; and
calibrating the predicted stress state value by using the calibration information, to obtain the theoretical stress state value of the user.

In this technical solution, the predicted stress state value output by the stress evaluation system is calibrated by using the calibration information, so that an obtained stress evaluation result is high in accuracy. In addition, the user does not need to give an assessment, so that user experience is improved.

A second aspect according to the invention provides a stress evaluation and calibration apparatus, including an obtaining module, a processing module, and a calibration module.

The obtaining module is configured to obtain a physiological parameter signal of a user.

The processing module is configured to: determine a smallest stress state value of the user within preset duration based on an eigenvalue vector of the physiological parameter signal and a stress evaluation system, and determine calibration information based on a smallest reference stress value of a group to which the user belongs and the smallest stress state value.

The calibration module is configured to calibrate, by using the calibration information, a stress state value output by the stress evaluation system, to determine a theoretical stress state value of the user.

According to the invention of the second aspect, the obtaining module is further configured to obtain an eigenvalue vector corresponding to the physiological parameter signal at each moment within the preset duration.

The processing module is specifically configured to: for the eigenvalue vector at each moment, input the eigenvalue vector into the stress evaluation system, to obtain a stress state value of the user at each moment, and determine the smallest stress state value of the user within the preset duration based on the stress state value of the user at each moment within the preset duration.

According to the invention, the eigenvalue vector includes a plurality of eigenvalue components, and the stress state value of the user at each moment is obtained by performing weighted summation based on each eigenvalue component in the eigenvalue vector and a weight value corresponding to each eigenvalue component.

In another possible implementation of the second aspect, the obtaining module is further configured to obtain basic information of the user before the processing module determines the calibration information based on the smallest reference stress value of the group to which the user belongs and the smallest stress state value.

The processing module is further configured to: determine a group identifier of the user based on the basic information, and query a stress value database based on the group identifier of the user, to determine the smallest reference stress value of the group to which the user belongs, where the stress value database stores a correspondence between a group identifier and a reference stress range.

In still another possible implementation of the second aspect, the calibration module is specifically configured to: input the eigenvalue vector of the physiological parameter signal into the stress evaluation system to obtain a predicted stress state value, and calibrate the predicted stress state value by using the calibration information, to obtain the theoretical stress state value of the user.

For beneficial technical effects that are not detailed in the possible implementations of the second aspect, refer to the descriptions in the first aspect. Details are not described herein again.

A third aspect of this application although not claimed provides a stress evaluation and calibration apparatus, including a processor, a memory, and a computer program that is stored in the memory and that can be run on the processor. When executing the program, the processor implements the method in the first aspect and the possible implementations of the first aspect.

A fourth aspect of this application provides a storage medium. The storage medium stores instructions, and when the instructions are run on a computer, the computer is enabled to perform the method in the first aspect and the possible implementations of the first aspect.

A fifth aspect of this application provides although not claimed a program product including instructions. When the program product runs on a computer, the computer is enabled to perform the method in the first aspect and the possible implementations of the first aspect.

A sixth aspect of this application although not claimed provides a chip. The chip includes a memory and a processor. The memory stores code and data, and is coupled to the processor. The processor runs the code in the memory, to enable the chip to perform the method in the first aspect and the possible implementations of the first aspect.

With the stress evaluation and calibration method and apparatus, and the storage medium that are provided in the embodiments of this application, the physiological parameter signal of the user is obtained. The smallest stress state value of the user within the preset duration is determined based on the eigenvalue vector of the physiological parameter signal and the stress evaluation system. The calibration information is determined based on the smallest reference stress value of the group to which the user belongs and the smallest stress state value. In addition, the stress state value output by the stress evaluation system is calibrated by using the calibration information, to determine the theoretical stress state value of the user. In this technical solution, the stress evaluation result can be automatically calibrated, to improve the evaluation accuracy, and the active participation of the user is not required, to improve user experience.

### BRIEF DESCRIPTION OF DRAWINGS

FIG. 1 is a schematic structural diagram of a stress evaluation and calibration system according to an embodiment of this application;
FIG. 2 is a schematic flowchart of Embodiment 1 of a stress evaluation and calibration method according to an embodiment of this application;
FIG. 3 is a schematic flowchart of Embodiment 2 of a stress evaluation and calibration method according to an embodiment of this application;
FIG. 4 is a schematic flowchart of Embodiment 3 of a stress evaluation and calibration method according to an embodiment of this application;
FIG. 5 is a schematic flowchart of Embodiment 4 of a stress evaluation and calibration method according to an embodiment of this application;
FIG. 6 is a schematic structural diagram of Embodiment 1 of a stress evaluation and calibration apparatus according to an embodiment of this application; and
FIG. 7 is a schematic structural diagram of Embodiment 2 of a stress evaluation and calibration apparatus according to an embodiment of this application.

### DESCRIPTION OF EMBODIMENTS

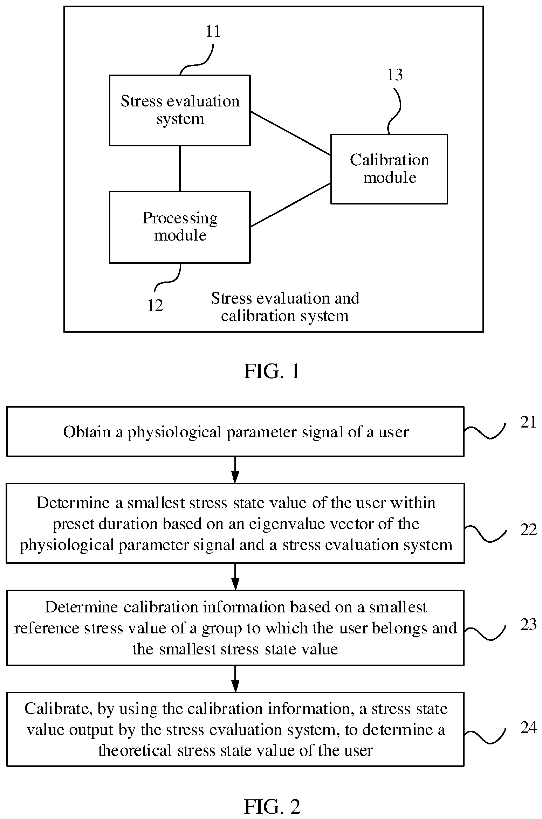

A stress evaluation and calibration method provided in the following embodiments of this application is applicable to a stress evaluation and calibration system. FIG. 1 is a schematic structural diagram of a stress evaluation and calibration system according to an embodiment of this application. As shown in FIG. 1, the stress evaluation and calibration system may include a stress evaluation system 11, a processing module 12, and a calibration module 13 that are connected to each other.

The stress evaluation system 11 may be a device with a stress assessment capability, and may obtain a physiological parameter signal of a user, analyze the physiological parameter signal, and output a user psychological stress value determined by the stress evaluation system. The processing module 12 may obtain a user stress value determined by the stress evaluation system 11, and process the user stress value based on an obtained reference stress value of a group to which the user belongs, to obtain calibration information of the user stress value. The calibration module 13 may obtain the user stress value determined by the stress evaluation system 11 and the calibration information obtained by the processing module 12, and calibrate the user stress value by using the calibration information, to obtain a theoretical stress state value.

Specific composition of the stress evaluation and calibration system is not limited in this embodiment of this application. The stress evaluation and calibration system may further include another module, for example, a storage module or a communications interface. The specific composition of the stress evaluation and calibration system may be limited based on an actual situation. Details are not described herein again.

In the embodiments of this application, "a plurality of" means two or more than two. The term "and/or" describes an association between associated objects and indicates that three relationships may exist. For example, A and/or B may indicate the following three cases: Only A exists, both A and B exist, and only B exists. The character "/" generally indicates an "or" relationship between the associated objects.

The following first briefly describes a scenario to which the embodiments of this application are applicable.

As social economy rapidly develops and pace of life accelerates, people withstand various kinds of stress at all times, and fierce competition makes mental health problems increasingly prominent. Psychological research shows that some events in life are main stress sources that lead to psychological stress and that harm health, and that there is an inverted U-shaped curve relationship between work performance and stress. Moderate stress can improve work efficiency, but a high level of stress very possibly leads to a "stress crisis", thereby affecting people's mental health.

Mental stress significantly affects people's work and life efficiency, quality of life, and the like. Long-term stress can induce occurrence of various diseases, for example, fatigability, memory loss, bad appetite, even a palpitation, difficult breathing, and an abdominal cramp. Therefore, comprehensive evaluation of people's stress state values may enable people to know people's stress levels in time, and comprehensively understand specific sources of people's stress, to obtain scientific, professional and targeted stress regulation schemes, so as to effectively maintain and promote people's physical and psychological health.

In addition, qualitative and quantitative assessment of mental stress of a user is also valuable to some extent. The assessment helps push sports and service commodities, assist in an early warning of a physical condition, and can remind the user of excessive stress. For example, a daily time period during which people are in a good mental state is analyzed. In this way, people can properly arrange work, to improve the work efficiency and the like.

With rise and portability of wearable devices, the wearable devices gradually become new carriers for mental stress assessment. More wearable devices may collect physiological parameter signals of people in a daily scenario, for example, a heart rate value and a skin temperature, to present the signals to people.

Currently, for a stress evaluation result of an existing stress evaluation system, the stress evaluation result is usually calibrated based on user self-evaluation information obtained through sampling before or after evaluation, to finally obtain an actual stress assessment result. However, in the foregoing calibration manner, the user self-evaluation information needs to be obtained in a manner that the user answers a question, and problems of poor user experience and subjective uncertainty exist.

To address the foregoing problems, the embodiments of this application provide a stress evaluation and calibration method. The method includes: obtaining a physiological parameter signal of a user; determining a smallest stress state value of the user within preset duration based on an eigenvalue vector of the physiological parameter signal and a stress evaluation system; determining calibration information based on a smallest reference stress value of a group to which the user belongs and the smallest stress state value; and calibrating, by using the calibration information, a stress state value output by the stress evaluation system, to determine a theoretical stress state value of the user. In this technical solution, a stress evaluation result can be automatically calibrated, to improve evaluation accuracy, and active participation of the user is not required, to improve user experience.

The following describes the technical solutions of this application in detail with reference to specific embodiments. It should be noted that the following several specific embodiments may be combined with each other, and a same or similar concept or process may not be described repeatedly in some embodiments.

FIG. 2 is a schematic flowchart of Embodiment 1 of the stress evaluation and calibration method according to the embodiments of this application. The method is applicable to the stress evaluation and calibration system shown in FIG. 1. The system may be implemented by a server, or may be implemented by another electronic device with evaluation and calibration capabilities. For example, the electronic device may be a wearable device such as a band or a smartwatch. Optionally, as shown in FIG. 2, the stress evaluation and calibration method may include the following steps.

Step 21: Obtain the physiological parameter signal of the user.

Optionally, in a daily scenario, there are a relatively large quantity of devices or apparatuses for physiological parameter signal collection, for example, a device such as a band or a smartwatch. Therefore, the device or the apparatus may collect the physiological parameter signal of the user, and assess and track a psychological stress value level of the user based on the collected physiological parameter signal.

For example, for an apparatus having a heart rate collector, for example, the band or the smartwatch, the apparatus may collect a heart rate value of the user, and further evaluate a stress state value of the user based on the heart rate value of the user, to assess and track psychological stress of the user based on a stress state value of the user within a preset time period.

In this embodiment, the server or the electronic device may obtain the physiological parameter signal of the user, and further process the physiological parameter signal. For example, the physiological parameter signal may include different physiological signals such as heart rate information, electrocardio information, blood pressure information, and weight information.

Specifically, in this embodiment, the server or the electronic device may obtain physiological parameter signals continuously collected by a device (for example, a wearable device) within the preset time period. The physiological parameter signals may include a pulse wave signal collected by using photoplethysmography (photoplethysmography, PPG), heart rate information obtained based on the pulse wave signal, or an electrocardio signal collected by using an electrocardiogram (electrocardiogram, ECG).

In this embodiment of this application, a specific parameter of the obtained physiological parameter signal may be determined based on an actual situation. Details are not described herein again.

Step 22: Determine the smallest stress state value of the user within the preset duration based on the eigenvalue vector of the physiological parameter signal and the stress evaluation system.

For example, in this embodiment, the server or the foregoing electronic device may analyze the obtained physiological parameter signal of the user, to obtain the eigenvalue vector of the physiological parameter signal. Similarly, a physiological parameter signal of the user at each moment within the preset duration may be analyzed, to obtain a corresponding eigenvalue vector of the physiological parameter signal of the user at each moment within the preset duration. For example, heart rate variability (heart rate variability, HRV) analysis is performed on the heart rate information to obtain an eigenvalue vector corresponding to the heart rate information, and spectrum analysis is performed on the electrocardio signal to obtain an eigenvalue vector corresponding to the electrocardio signal.

In this embodiment, the stress evaluation system may be an existing device or apparatus with a stress evaluation capability. The corresponding eigenvalue vector of the physiological parameter signal at each moment is input into the stress evaluation system. The stress evaluation system may obtain a stress state value at each moment, and may obtain the smallest stress state value of the user within the preset duration by comparing the stress state values at all the moments.

Step 23: Determine the calibration information based on the smallest reference stress value of the group to which the user belongs and the smallest stress state value.

Optionally, for a wearable device that can collect the physiological parameter signal of the user, before using the wearable device, the user may first collect basic information of the user, such as a height, a weight, and a gender. When the user uses the wearable device, the wearable device may further collect other basic information of the user, such as sleep duration and sleep quality. Therefore, the group to which the user belongs may be determined based on the basic information of the user, for example, infants, children, adolescents, middle-aged persons, or elderly persons. Finally, the smallest reference stress value of the group to which the user belongs is determined based on a reference stress value range of the group to which the user belongs.

In this embodiment, with reference to the smallest stress state value of the user within the preset duration determined in step 22 and the smallest reference stress value of the group to which the user belongs, the smallest stress state value of the user within the preset duration is matched with the smallest reference stress value of the group to which the user belongs, to determine a difference between the smallest reference stress value and the smallest stress state value. Further, the difference is used as the calibration information of the stress evaluation system.

Optionally, the calibration information may be used to calibrate a subsequent stress result, a stress result of a user in a previous period of time, or the stress evaluation system. For a specific application manner of the calibration information, refer to descriptions in the following step 24. Details are not described herein.

It should be noted that, in this embodiment, the smallest stress state value in this embodiment may be a smallest value determined after vector sorting is performed on the stress state values at all the moments within the preset duration, a stress state value collected at a moment at which the user is most relaxed within a day, or a smallest stress state value of the user during a deep sleep period. A specific manner of obtaining the smallest stress state value of the user within the preset duration is not limited in this embodiment of this application, and may be determined based on an actual situation.

Step 24: Calibrate, by using the calibration information, the stress state value output by the stress evaluation system, to determine the theoretical stress state value of the user.

For example, in this embodiment, after the calibration information is determined, a calibration module may be connected to an output part of the stress evaluation system, and a calibration function of the calibration module may be obtained by using the calibration information. In this way, after the stress state value output by the stress evaluation system is calibrated by using the calibration module, the theoretical stress state value of the user may be obtained.

In a possible design of this embodiment, the electronic device or the server may alternatively process the eigenvalue vector of the physiological parameter signal by using the calibration information, and then input the processed eigenvalue vector into the stress evaluation system, so that the stress evaluation system outputs the theoretical stress state value of the user.

In another possible design of this embodiment, the electronic device or the server may alternatively input both the calibration information and an eigenvalue vector of a physiological parameter signal at a moment corresponding to the smallest stress state value into the stress evaluation system, to update a parameter of the stress evaluation system, so that an output of the stress evaluation system is infinitely close to or equal to the smallest reference stress value. Therefore, after the physiological parameter signal of the user is obtained, the physiological parameter signal may be directly input into the stress evaluation system, to obtain the theoretical stress state value of the user.

It should be noted that an actual operation manner of calibrating an output result of the stress evaluation system by using the calibration information is not limited in this embodiment of this application, and may be determined based on an actual situation. Details are not described herein again.

In this embodiment, the user does not need to provide a self-evaluation comment, and the calibration information may be automatically generated, to automatically calibrate the stress evaluation result. A self-learning and real-time update mechanism is provided. In other words, user-unaware stress calibration is implemented, and better balance between stress evaluation accuracy and user experience is implemented.

According to the stress evaluation and calibration method provided in this embodiment of this application, the physiological parameter signal of the user is obtained. The smallest stress state value of the user within the preset duration is determined based on the eigenvalue vector of the physiological parameter signal and the stress evaluation system. The calibration information is determined based on the smallest reference stress value of the group to which the user belongs and the smallest stress state value. In addition, the stress state value output by the stress evaluation system is calibrated by using the calibration information, to determine the theoretical stress state value of the user. In this technical solution, the user does not need to provide the self-evaluation comment, and the calibration information may be automatically generated, to automatically calibrate the stress evaluation result. This improves the evaluation accuracy. In addition, the active participation of the user is not required, to improve user experience.

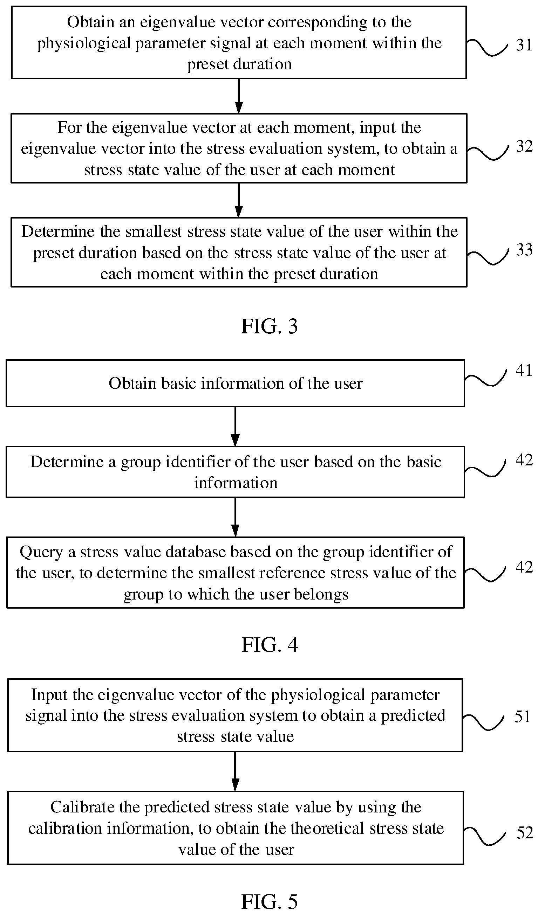

For example, based on the foregoing embodiment, FIG. 3 is a schematic flowchart of Embodiment 2 of the stress evaluation and calibration method according to the embodiments of this application. As shown in FIG. 3, step 22 may be implemented by using the following steps.

Step 31: Obtain the eigenvalue vector corresponding to the physiological parameter signal at each moment within the preset duration.

The eigenvalue vector includes at least one eigenvalue component.

Optionally, in this embodiment, after obtaining the physiological parameter signal, the electronic device (for example, the wearable device) or the server may perform feature extraction on the physiological parameter signal, to obtain the eigenvalue vector corresponding to the physiological parameter signal of the user at each moment, and correspondingly store the eigenvalue vector. The eigenvalue vector of the physiological parameter signal may include a time domain feature indicator and a frequency domain feature indicator.

For example, for a j^{th} moment within the preset duration, it is assumed that an eigenvalue vector corresponding to the physiological parameter signal of the user is *v_j* = {*v_*1*j,v_*2*j,v_*3*j,*...,*v_nj* }, where *v_j* represents the eigenvalue vector at the j^{th} moment, and *v*_*nj* represents an n^{th} eigenvalue component in the eigenvalue vector at the j^{th} moment.

For example, when the physiological parameter signal is heart rate information, an eigenvalue vector of the heart rate information includes a time domain feature indicator and a frequency domain feature indicator that are obtained by using the heart rate variability analysis. Optionally, the eigenvalue vector of the heart rate information may include eigenvalue components such as a total power (total power, TP) spectrum, a high frequency (high frequency, HF) band, and a low frequency (low frequency, LF) band of a heart rate spectrum curve in the frequency domain feature indicator, or a standard deviation of NN intervals (standard deviation of NN intervals, SDNN) in the time domain feature indicator.

The LF band reflects dual regulation of a sympathetic nerve and a vagus nerve. The HF band reflects only regulation of the vagus nerve. The TP reflects magnitude of HRV. The SDNN is used to evaluate magnitude of a total heart rate change. The NN interval may be a preset time period.

For example, for eigenvalue vectors including the eigenvalue components such as the TP spectrum, the HF band, the LF band, and the SDNN, an eigenvalue vector ***v*_1** of the physiological parameter signal at a 1^{st} moment may be represented as ***v*_1** = {TP = 1.1, HF = 2.0, LF = 3.0, SDNN = 3.1}, an eigenvalue vector ***v*_2** at a 2^{nd} moment may be represented as ***v*_2** = {TP = 1.2, HF = 1.0, LF = 2.0, SDNN = 1.1}, and an eigenvalue vector ***v*_3** at a 3^{rd} moment may be represented as ***v*_3** = {TP = 1.5, HF = 3.0, LF = 6.0, SDNN = 0}. Similarly, an eigenvalue vector at another moment may be represented in a same manner.

It should be noted that, in this embodiment of this application, the eigenvalue vector of the heart rate information is not limited to including the foregoing frequency domain feature indicator and time domain feature indicator, and may further include another time domain feature indicator and another frequency domain feature indicator.

For example, the time domain feature indicator may further include an HRV triangular index, a standard deviation of average NN intervals (SDANN), and a root mean square of successive differences in adjacent NN intervals (RMSSD). The HRV triangular index is also used to evaluate the magnitude of the total heart rate change. The SDANN is used to evaluate a long-term slow-changing component in a heart rate change. The RMSSD reflects magnitude of a fast-changing component in the heart rate change.

The frequency domain feature indicator may further include a very low frequency (VLF) band and an LF/HF ratio. The VLF band reflects impact of heat regulation (a body temperature), vasomotor tension and a renin-angiotensin system on the heart rate change. The LF/HF ratio reflects a balance state of an autonomic nervous system, and basically represents a tension level of the sympathetic nerve.

Step 32: For the eigenvalue vector at each moment, input the eigenvalue vector into the stress evaluation system, to obtain the stress state value of the user at each moment.

Optionally, in this embodiment, the stress evaluation system may be obtained through training based on a relationship between the physiological parameter signal (for example, obtained by using a physiological parameter signal sensor) and the stress state value of the user (obtained by using a psychological assessment table). Therefore, the stress evaluation system has a function of determining a stress state value based on an eigenvalue vector of a physiological parameter signal.

Correspondingly, for the eigenvalue vector at each moment, the eigenvalue vector may be input into the stress evaluation system, and correspondingly, the stress evaluation system may output a stress state value of the user at the current moment.

In this embodiment, the stress evaluation system may be locally stored. In this way, when the physiological parameter signal of the user is obtained, the stress evaluation model may be directly used to perform stress evaluation and operated anytime and anywhere. Stress evaluation is easily implemented. The stress evaluation system may alternatively be stored in a cloud server. In this way, not only can occupation of a local memory be reduced, but also a data volume of the stress evaluation system in the cloud server can be enriched, to update a general-purpose stress evaluation system. A specific storage location of the stress evaluation system is not limited in this embodiment of this application, and may be determined based on an actual situation.

It should be noted that the stress state value of the user at each moment is obtained by performing weighted summation based on each eigenvalue component in the eigenvalue vector and a weight value corresponding to each eigenvalue component.

Specifically, for a physiological parameter signal, in a training process of the stress evaluation model, a weight value corresponding to each eigenvalue component in an eigenvalue vector may be determined based on a contribution value of each eigenvalue component to a stress state value. Therefore, for the eigenvalue vector at each moment, a weighted summation operation may be performed on each eigenvalue component in the eigenvalue vector at the current moment and the corresponding weight value, to obtain the stress state value of the user at the current moment.

In this embodiment, for the eigenvalue vector at the j^{th} moment, a stress state value at the j^{th} moment is equal to y_j = v_1j _{*} m_1 + v_2j _{*} m_2 + ··· + *v_nj* _{*} m_n, where m_n is a weight value of the n^{th} eigenvalue component.

For example, in this embodiment, it is assumed that m_1 = 0.1, m_2 = 0.2, m_3 = 0.5, m_4 = 0.2, the eigenvalue vector at the first moment is ***v*_1** = {TP = 1.1, HF = 2.0, LF = 3.0, SDNN = 3.1}, the eigenvalue vector at the 2^{nd} moment is ***v*_2** = {TP = 1.2, HF = 1.0, LF = 2.0, SDNN = 1.1}, and the eigenvalue vector at the 3^{rd} moment is ***v*_3** = {TP = 1.5, HF = 3.0, LF = 6.0, SDNN = 0}. Therefore, based on a formula y_j = v_1j _{*} m_1 + v_2j _{*} m_2 + ··· + *v_nj* _{*} m_n, a stress state value y_1 = 0.1 _{*} 1.1 + 0.2 _{*} 2.0 + 0.5 _{*} 3.0 + 0.2 _{*} 3.1 = 2.63 at the first moment, a stress state value y_2 = 0.1 _{*} 1.2 + 0.2 _{*} 1.0 + 0.5 _{*} 2.0 + 0.2 _{*} 1.1 = 1.54 at the second moment, and a stress state value y_3 = 0.1 _{*} 1.5 + 0.2 _{*} 3.0 + 0.5 _{*} 6.0 + 0.2 _{*} 0 = 3.75 at the third moment may be obtained.

Step 33: Determine the smallest stress state value of the user within the preset duration based on the stress state value of the user at each moment within the preset duration.

Optionally, in this embodiment, after the stress state value of the user at each moment is obtained in step 32, a smallest stress state value may be determined by comparing the stress state values one by one. Alternatively, the stress state values at all the moments may be sorted in a preset order (in ascending order or descending order), to obtain the smallest stress state value.

For example, for heart rate information of the user, it can be learned from step 31 and step 32 that the stress state values at the foregoing three moments are respectively as follows: y_1 = 2.63, y_2 = 1.54, and y_3 = 3.75. It can be learned through comparison or sorting that a smallest stress state value is y_2 = 1.54, and a corresponding eigenvalue vector is v_2 = {TP = 1.2, HF = 1.0, LF = 2.0, SDNN = 1.1}.

According to the stress evaluation and calibration method provided in this embodiment of this application, the eigenvalue vector corresponding to the physiological parameter signal at each moment within the preset duration is obtained. For the eigenvalue vector at each moment, the eigenvalue vector is input into the stress evaluation system, to obtain the stress state value of the user at each moment. Finally, the smallest stress state value of the user within the preset duration is determined based on the stress state value of the user at each moment within the preset duration. In this technical solution, the smallest stress state value of the user within the preset duration, namely, the stress state value at the most relaxed moment, may be determined based on the eigenvalue vector of the physiological parameter signal. This provides an implementation possibility for subsequently determining the calibration information.

For example, based on any one of the foregoing embodiments, FIG. 4 is a schematic flowchart of Embodiment 3 of the stress evaluation and calibration method according to the embodiments of this application. As shown in FIG. 4, before step 23, the method may further include the following steps.

Step 41: Obtain the basic information of the user.

The basic information includes the height, the weight, the gender, and an age.

Generally, before being used by the user, the wearable device may collect the basic information of the user, for example, the gender, the height, the weight, and the age. In addition, in a process in which the wearable device is used by the user, the wearable device may further obtain the other information of the user, for example, the sleep duration and the sleep quality. In this way, when a stress state of the user needs to be evaluated, the electronic device, the wearable device, or the server may first obtain the basic information of the user. This lays a foundation for subsequently determining the reference stress range of the group to which the user belongs.

It should be noted that the obtained basic information of the user is not limited in this embodiment of this application. For example, the basic information may alternatively be age information, work and rest information, travel information, occupation information, or other basic information of the user. Content included in the basic information of the user may be determined based on an actual situation, and details are not described herein again.

Step 42: Determine a group identifier of the user based on the basic information.

Optionally, for users having different heights, weights, genders, and ages, the users are classified into different groups, for example, the infants, the children, the adolescents, the middle-aged persons, and the elderly persons. Correspondingly, each group has a corresponding group identifier.

For example, in this embodiment, based on age information, work and rest information, travel information, occupation information, or other basic information, people participating in a survey may also be classified into different groups, for example, scientific and technical personnel, medical personnel, teachers, students, and freelancers. Correspondingly, each group has a corresponding group identifier.

Therefore, in this embodiment, the group identifier of the user may be determined based on the obtained basic information of the user.

Step 43: Query a stress value database based on the group identifier of the user, to determine the smallest reference stress value of the group to which the user belongs.

The stress value database stores a correspondence between a group identifier and a reference stress range.

Optionally, for different groups, stress state values of different users within a preset time period may be surveyed and tracked by using questionnaires. Alternatively, stress state values of different users within a preset time period may be determined based on subjective self-evaluation of the users. The stress state values of all the users participating in the survey are combined, to determine reference stress ranges corresponding to the different groups. Correspondingly, different group identifiers and reference stress ranges corresponding to the group identifiers may be stored in the stress value database, to determine a smallest reference stress value of an evaluated user.

Optionally, the stress value database may be stored in the cloud server. In this way, not only can the occupation of the local memory be reduced, but also the data volume of the stress value database in the cloud server can be enriched, so as to update the stress value database. The stress value database may alternatively be stored locally. In this way, when the group identifier of the user is determined, the smallest reference stress value of the group to which the user belongs may be queried based on the group identifier of the user. A response speed is relatively fast. An operation can be performed anytime and anywhere. For example, the operation can still be performed in a place at which there is no network, to obtain the smallest reference stress value of the group to which the user belongs.

It should be noted that the smallest reference stress value may be a value, a range, or the like. The smallest reference pressure value may be determined based on an actual situation, and this is not limited in this embodiment.

According to the stress evaluation and calibration method provided in this embodiment of this application, the basic information of the user is obtained. The group identifier of the user is determined based on the basic information. The stress value database is queried based on the group identifier of the user, to determine the smallest reference stress value of the group to which the user belongs. In this technical solution, the smallest reference stress value of the group to which the user belongs is determined. In this way, the calibration information of the stress evaluation system may be automatically determined. In addition, the active participation of the user is not required, so that user experience is improved.

Further, based on any one of the foregoing embodiments, FIG. 5 is a schematic flowchart of Embodiment 4 of the stress evaluation and calibration method according to the embodiments of this application. As shown in FIG. 5, step 24 may be implemented by using the following steps.

Step 51: Input the eigenvalue vector of the physiological parameter signal into the stress evaluation system to obtain a predicted stress state value.

Optionally, because the stress evaluation system has a stress evaluation function, but accuracy is not high, after the physiological parameter signal is obtained, the eigenvalue vector of the physiological parameter signal may be first input into the stress evaluation system, to obtain the predicted stress state value output by the stress evaluation system.

For example, it can be learned from the embodiment shown in FIG. 3 that, for the heart rate information, a first predicted stress state value of the user within the preset time period is y_1 = 2.63, a corresponding eigenvalue vector is ***v*_1** = {TP = 1.1, HF = 2.0, LF = 3.0, SDNN = 3.1}; a second predicted stress state value is y_2 = 1.54, a corresponding eigenvalue vector is v_2 = {TP = 1.2, HF = 1.0, LF = 2.0, SDNN = 1.1}; and a third predicted stress state value is y_3 = 3.75, and a corresponding eigenvalue vector is v_3 = {TP = 1.5, HF = 3.0, LF = 6.0, SDNN = 0}.

Step 52: Calibrate the predicted stress state value by using the calibration information, to obtain the theoretical stress state value of the user.

In this embodiment, it can be learned from step 23 in the embodiment shown in FIG. 2 that the difference between the smallest reference stress value of the group to which the user belongs and the smallest stress state value of the user within the preset duration may be used as the calibration information of the stress evaluation system. Therefore, for the heart rate information of the user, if the smallest reference stress value of the group to which the user belongs is Y_min = 15, and the smallest stress state value is y_2 = 1.54, the calibration information of the stress evaluation system may be represented as Y_min - y_2 = 15 - 1.54 = 13.46.

Therefore, in this embodiment, the theoretical stress state value of the user may be equal to a sum of the predicted stress state value and the calibration information. In other words, a theoretical stress state value corresponding to the first predicted stress state value y_1 = 2.63 is y_1 + the calibration information = 2.63 + 13.46 = 16.09, and a theoretical stress state value corresponding to the third predicted stress state value y_3 = 3.75 is y_3 + the calibration information = 3.75 + 13.46 = 17.21.

It should be noted that the stress state value in this embodiment of this application may range from 0 to 100, but this is not limited in this embodiment of this application.

According to the stress evaluation and calibration method provided in this embodiment of this application, the eigenvalue vector of the physiological parameter signal is input into the stress evaluation system to obtain the predicted stress state value, and the predicted stress state value is calibrated by using the calibration information, to obtain the theoretical stress state value of the user. In this technical solution, the predicted stress state value output by the stress evaluation system is calibrated by using the calibration information, so that an obtained stress evaluation result is high in accuracy. In addition, the user does not need to give an assessment, so that user experience is improved.

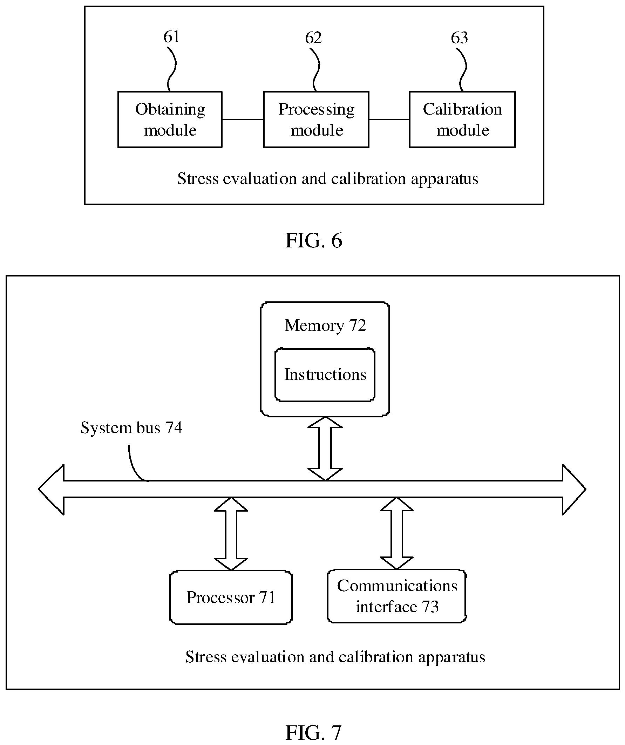

FIG. 6 is a schematic structural diagram of Embodiment 1 of a stress evaluation and calibration apparatus according to an embodiment of this application. The apparatus may be integrated into an electronic device or a server, or may be implemented by using an electronic device or a server. As shown in FIG. 6, the apparatus may include an obtaining module 61, a processing module 62, and a calibration module 63.

The obtaining module 61 is configured to obtain a physiological parameter signal of a user.

The processing module 62 is configured to: determine a smallest stress state value of the user within preset duration based on an eigenvalue vector of the physiological parameter signal and a stress evaluation system, and determine calibration information based on a smallest reference stress value of a group to which the user belongs and the smallest stress state value.

The calibration module 63 is configured to calibrate, by using the calibration information, a stress state value output by the stress evaluation system, to determine a theoretical stress state value of the user.

For example, in a possible design of this embodiment of this application, the obtaining module 61 is further configured to obtain an eigenvalue vector corresponding to the physiological parameter signal at each moment within the preset duration.

The processing module 62 is specifically configured to: for the eigenvalue vector at each moment, input the eigenvalue vector into the stress evaluation system, to obtain a stress state value of the user at each moment, and determine the smallest stress state value of the user within the preset duration based on the stress state value of the user at each moment within the preset duration.

Optionally, in this embodiment, the eigenvalue vector includes at least one eigenvalue component, and the stress state value of the user at each moment is obtained by performing weighted summation based on each eigenvalue component in the eigenvalue vector and a weight value corresponding to each eigenvalue component.

For example, in another possible design of this embodiment of this application, the obtaining module 61 is further configured to obtain basic information of the user before the processing module 62 determines the calibration information based on the smallest reference stress value of the group to which the user belongs and the smallest stress state value.

The processing module 62 is further configured to: determine a group identifier of the user based on the basic information, and query a stress value database based on the group identifier of the user, to determine the smallest reference stress value of the group to which the user belongs, where the stress value database stores a correspondence between a group identifier and a reference stress range.

For example, in still another possible design of this embodiment of this application, the calibration module 63 is specifically configured to: input the eigenvalue vector of the physiological parameter signal into the stress evaluation system to obtain a predicted stress state value, and calibrate the predicted stress state value by using the calibration information, to obtain the theoretical stress state value of the user.

The stress evaluation and calibration apparatus in this embodiment may be configured to execute the implementation solutions of the method embodiments shown in FIG. 2 to FIG. 5. Specific implementations and technical effects are similar, and details are not described herein again.

It should be noted and understood that division into the modules of the foregoing apparatus is merely logic function division. In an actual implementation, some or all modules may be integrated into one physical entity, or the modules may be physically separated. In addition, all these modules may be implemented in a form of software invoked by a processor element, or may be implemented in a form of hardware. Alternatively, some modules may be implemented in a form of software invoked by a processor element, and some modules are implemented in a form of hardware. For example, a determining module may be an independently disposed processor element, or may be integrated in a chip of the foregoing apparatus for implementation. In addition, the determining module may alternatively be stored in a memory of the foregoing apparatus in a form of program code and invoked by a processor element of the foregoing apparatus to perform a function of the determining module. An implementation of another module is similar to the implementation of the determining module. In addition, all or some of these modules may be integrated together, or may be implemented independently. The processor element described herein may be an integrated circuit, and has a signal processing capability. In an implementation process, steps in the foregoing methods or the foregoing modules can be implemented by using a hardware integrated logic circuit in the processor element, or by using instructions in a form of software.

For example, the foregoing modules may be configured as one or more integrated circuits for implementing the foregoing methods, such as one or more application-specific integrated circuits (application-specific integrated circuits, ASICs), one or more microprocessors (microprocessors), or one or more field programmable gate arrays (field programmable gate arrays, FPGAs). For another example, when one of the foregoing modules is implemented in a form of invoking program code by a processor element, the processor element may be a general-purpose processor, for example, a central processing unit (central processing unit, CPU) or another processor that can invoke the program code. For another example, these modules may be integrated together and implemented in a form of a system-on-a-chip (system-on-a-chip, SOC).

All or some of the foregoing embodiments may be implemented by using software, hardware, firmware, or any combination thereof. When software is used to implement the embodiments, all or some of the foregoing embodiments may be implemented in a form of a computer program product. The computer program product includes one or more computer instructions. When the computer instructions are loaded and executed on a computer, the procedure or functions according to the embodiments of this application are completely or partially generated. The computer may be a general-purpose computer, a dedicated computer, a computer network, or another programmable apparatus. The computer instructions may be stored in a readable storage medium or may be transmitted from a readable storage medium to another readable storage medium. For example, the computer instructions may be transmitted from a website, computer, server, or data center to another website, computer, server, or data center in a wired (for example, a coaxial cable, an optical fiber, or a digital subscriber line (DSL)) or wireless (for example, infrared, radio, or microwave) manner. The readable storage medium may be any usable medium accessible by the computer, or a data storage device, such as a server or a data center, integrating one or more usable media. The usable medium may be a magnetic medium (for example, a floppy disk, a hard disk, or a magnetic tape), an optical medium (for example, a DVD), a semiconductor medium (for example, a solid-state drive (solid-state drive, SSD)), or the like.

FIG. 7 is a schematic structural diagram of Embodiment 2 of a stress evaluation and calibration apparatus according to an embodiment of this application. As shown in FIG. 7, the apparatus may include a processor 71, a memory 72, a communications interface 73, and a system bus 74. The memory 72 and the communications interface 73 are connected to the processor 71 and communicate with each other through the system bus 74. The memory 72 is configured to store a computer program. The communications interface 73 is configured to communicate with another device. When executing the computer program, the processor 71 implements the methods in the embodiments shown in FIG. 2 to FIG. 5.

The system bus mentioned in FIG. 7 may be a peripheral component interconnect (peripheral component interconnect, PCI) bus, an extended industry standard architecture (extended industry standard architecture, EISA) bus, or the like. The system bus may be classified into an address bus, a data bus, a control bus, and the like. For ease of representation, only one thick line is used to represent the bus in the figure, but this does not mean that there is only one bus or only one type of bus. The communications interface is configured to implement communication between a database access apparatus and another device (such as a client, a read/write database, or a read-only database). The memory may include a random access memory (random access memory, RAM), or may be a non-volatile memory

(non-volatile memory), for example, at least one magnetic disk memory.

The processor may be a general-purpose processor, including a central processing unit (Central Processing Unit, CPU), a network processor (Network Processor, NP), or the like; or may be a digital signal processor (Digital Signal Processor, DSP), an application-specific integrated circuit (Application-Specific Integrated Circuit, ASIC), a field programmable gate array (Field Programmable Gate Array, FPGA) or another programmable logic device, a discrete gate or a transistor logic device, a discrete hardware component, or the like.

Optionally, an embodiment of this application further provides a storage medium. The storage medium stores instructions, and when the instructions are run on a computer, the computer is enabled to perform the methods in the embodiments shown in FIG. 2 to FIG. 5.

Optionally, an embodiment of this application further provides a chip for running instructions. The chip is configured to perform the methods in the embodiments shown in FIG. 2 to FIG. 5.

An embodiment of this application further provides a program product. The program product includes a computer program, the computer program is stored in a storage medium, and at least one processor may read the computer program from the storage medium. When executing the computer program, the at least one processor may implement the methods in the embodiments shown in FIG. 2 to FIG. 5.

In this application, "at least one" means one or more, and "a plurality of" means two or more than two. The term "and/or" describes an association between associated objects and indicates that three relationships may exist. For example, A and/or B may indicate the following cases: Only A exists, both A and B exist, and only B exists. A and B may be singular or plural. The character "/" generally indicates an "or" relationship between the associated objects. In a formula, the character "/" indicates a "division" relationship between the associated objects. "At least one of the following items (pieces)" or a similar expression thereof means any combination of these items, including any combination of singular items (pieces) or plural items (pieces). For example, at least one of a, b, or c may indicate: a, b, c, a and b, a and c, b and c, or a, b, and c, where a, b, and c may be singular or plural.

It may be understood that various numbers in the embodiments of this application are merely used for differentiation for ease of description, and are not used to limit the scope of the embodiments of this application.

It should be understood that sequence numbers of the foregoing processes do not mean execution sequences in the embodiments of this application. The execution sequences of the processes should be determined according to functions and internal logic of the processes, and should not be construed as any limitation on the implementation processes of the embodiments of this application.

## Claims

1. A stress evaluation and calibration method carried out by a stress evaluation and calibration apparatus comprising an obtaining module (61), a processing module (12, 62), and a calibration module (13, 63), said method comprising:
obtaining (21), by the obtaining module (61), a physiological parameter signal of a user;
determining (22), by the processing module (12, 62), a smallest stress state value of the user within preset duration based on an eigenvalue vector of the physiological parameter signal;
determining (23), by the processing module (12, 62), calibration information based on a smallest reference stress value of a group to which the user belongs and the smallest stress state value; and
calibrating (24), by the calibration module (13, 63), by using the calibration information, a stress state value, to determine a theoretical stress state value of the user;
wherein the determining a smallest stress state value of the user within preset duration based on an eigenvalue vector of the physiological parameter signal comprises:
obtaining (31) an eigenvalue vector corresponding to the physiological parameter signal at each moment within the preset duration;
for the eigenvalue vector at each moment, obtaining (32) a stress state value of the user at each moment based on the eigenvalue vector; and
determining (33) the smallest stress state value of the user within the preset duration based on the stress state value of the user at each moment within the preset duration; and
wherein the eigenvalue vector comprises a plurality of different eigenvalue components, and the stress state value of the user at each moment is obtained by performing weighted summation based on each eigenvalue component in the eigenvalue vector and a weight value corresponding to each eigenvalue component.

2. The method according to claim 1, wherein before the determining calibration information based on a smallest reference stress value of a group to which the user belongs and the smallest stress state value, the method further comprises:
obtaining (41) basic information of the user;
determining (42) a group identifier of the user based on the basic information; and
querying (42) a stress value database based on the group identifier of the user, to determine the smallest reference stress value of the group to which the user belongs, wherein the stress value database stores a correspondence between a group identifier and a reference stress range.

3. The method according to any one of claims 1 to 2, wherein the calibrating, by using the calibration information, a stress state value, to determine a theoretical stress state value of the user comprises:
obtaining (51) a predicted stress state value based on the eigenvalue vector; and
calibrating (52) the predicted stress state value by using the calibration information, to obtain the theoretical stress state value of the user.

4. A stress evaluation and calibration apparatus, comprising an obtaining module (61), a processing module (12, 62), and a calibration module (13, 63), wherein
the obtaining module (61) is configured to obtain (21) a physiological parameter signal of a user;
the processing module (12, 62) is configured to: determine (22) a smallest stress state value of the user within preset duration based on an eigenvalue vector of the physiological parameter signal, and determine (23) calibration information based on a smallest reference stress value of a group to which the user belongs and the smallest stress state value; and
the calibration module (13, 63) is configured to calibrate'(23), by using the calibration information, a stress state value, to determine a theoretical stress state value of the user;
wherein the obtaining module (61) is further configured to obtain an eigenvalue vector corresponding to the physiological parameter signal at each moment within the preset duration; and
the processing module (62) is specifically configured to: for the eigenvalue vector at each moment, obtain a stress state value of the user at each moment based on the eigenvalue vector, and determine the smallest stress state value of the user within the preset duration based on the stress state value of the user at each moment within the preset duration; and
wherein the eigenvalue vector comprises a plurality of different eigenvalue components, and the stress state value of the user at each moment is obtained by performing weighted summation based on each eigenvalue component in the eigenvalue vector and a weight value corresponding to each eigenvalue component.

5. The apparatus according to any one of claim 4, wherein the obtaining module (61) is further configured to obtain basic information of the user before the processing module (12, 62) determines the calibration information based on the smallest reference stress value of the group to which the user belongs and the smallest stress state value; and
the processing module (12, 62) is further configured to: determine a group identifier of the user based on the basic information, and query a stress value database based on the group identifier of the user, to determine the smallest reference stress value of the group to which the user belongs, wherein the stress value database stores a correspondence between a group identifier and a reference stress range.

6. The apparatus according to any one of claim 4 or 5, wherein the calibration module (63) is specifically configured to: obtain a predicted stress state value based on the eigenvalue vector, and calibrate the predicted stress state value by using the calibration information, to obtain the theoretical stress state value of the user.

7. A computer-readable storage medium, wherein the storage medium comprises instructions, which, when run on a computer, cause the computer is to perform the method according to any one of claims 1 to 3.

## Patentansprüche

1. Verfahren zur Stressbeurteilung und -kalibrierung, das von einer Vorrichtung zur Stressbeurteilung und -kalibrierung durchgeführt wird, die ein Einholungsmodul (61), ein Verarbeitungsmodul (12, 62) und ein Kalibrierungsmodul (13, 63) umfasst, wobei das Verfahren Folgendes umfasst:
Einholen (21) eines physiologischen Parametersignals eines Benutzers durch das Einholungsmodul (61);
Bestimmen (22) eines kleinsten Stresszustandswerts des Benutzers innerhalb einer voreingestellten Dauer durch das Verarbeitungsmodul (12, 62) basierend auf einem Eigenwertvektor des physiologischen Parametersignals;
Bestimmen (23) von Kalibrierungsinformationen durch das Verarbeitungsmodul (12, 62) basierend auf einem kleinsten Referenzstresswert einer Gruppe, zu der der Benutzer gehört, und auf dem kleinsten Stresszustandswert; und
Kalibrieren (24) eines Stresszustandswerts durch das Kalibrierungsmodul (13, 63) unter Verwendung der Kalibrierungsinformationen, um einen theoretischen Stresszustandswert des Benutzers zu bestimmen;
wobei das Bestimmen eines kleinsten Stresszustandswerts des Benutzers innerhalb einer voreingestellten Dauer basierend auf einem Eigenwertvektor des physiologischen Parametersignals Folgendes umfasst:
Einholen (31) eines Eigenwertvektors, der dem physiologischen Parametersignal zu jedem Zeitpunkt innerhalb der voreingestellten Dauer entspricht;
Einholen (32), für den Eigenwertvektor zu jedem Zeitpunkt, eines Stresszustandswerts des Benutzers zu jedem Zeitpunkt basierend auf dem Eigenwertvektor; und
Bestimmen (33) des kleinsten Stresszustandswerts des Benutzers innerhalb der voreingestellten Dauer basierend auf dem Stresszustandswert des Benutzers zu jedem Zeitpunkt innerhalb der voreingestellten Dauer; und
wobei der Eigenwertvektor eine Vielzahl von unterschiedlichen Eigenwertkomponenten umfasst und der Stresszustandswert des Benutzers zu jedem Zeitpunkt durch Durchführen einer gewichteten Summierung basierend auf jeder Eigenwertkomponente im Eigenwertvektor und einem jeder Eigenwertkomponente entsprechenden Gewichtungswert eingeholt wird.

2. Verfahren nach Anspruch 1, wobei das Verfahren vor dem Bestimmen von Kalibrierungsinformationen basierend auf einem kleinsten Referenzspannungswert einer Gruppe, zu der der Benutzer gehört, und dem kleinsten Stresszustandswert ferner Folgendes umfasst:
Erlangen (41) grundlegender Informationen des Benutzers;
Bestimmen (42) einer Gruppenkennung des Benutzers basierend auf den Basisinformationen; und
Abfragen (42) einer Stresswertdatenbank basierend auf der Gruppenkennung des Benutzers, um den kleinsten Referenzstresswert der Gruppe zu bestimmen, zu der der Benutzer gehört, wobei die Stresswertdatenbank eine Entsprechung zwischen einer Gruppenkennung und einem Referenzstressbereich speichert.

3. Verfahren nach einem der Ansprüche 1 bis 2, wobei das Kalibrieren eines Stresszustandswerts unter Verwendung der Kalibrierungsinformationen zum Bestimmen eines theoretischen Stresszustandswerts des Benutzers Folgendes umfasst:
Erlangen (51) eines vorhergesagten Stresszustandswerts basierend auf dem Eigenwertvektor; und
Kalibrieren (52) des vorhergesagten Stresszustandswerts unter Verwendung der Kalibrierungsinformationen, um den theoretischen Stresszustandswert des Benutzers einzuholen.

4. Vorrichtung zur Stressbewertung und -kalibrierung, umfassend ein Einholungsmodul (61), ein Verarbeitungsmodul (12, 62) und ein Kalibrierungsmodul (13, 63), wobei das Einholungsmodul (61) zum Einholen (21) eines physiologischen Parametersignals eines Benutzers konfiguriert ist;
das Verarbeitungsmodul (12, 62) konfiguriert ist zum:
Bestimmen (22) eines kleinsten Stresszustandswerts des Benutzers innerhalb einer voreingestellten Dauer basierend auf einem Eigenwertvektor des physiologischen Parametersignals und Bestimmen (23) von Kalibrierungsinformationen basierend auf einer kleinsten Referenzstresswert einer Gruppe, zu der der Benutzer gehört, und dem kleinsten Stresszustandswert; und
das Kalibrierungsmodul (13, 63) zum Kalibrieren(23) eines Stresszustandswerts unter Verwendung der Kalibrierungsinformationen konfiguriert ist, um einen theoretischen Stresszustandswert des Benutzers zu bestimmen;
wobei das Einholungsmodul (61) ferner zum Einholen eines Eigenvektors, der dem physiologischen Parametersignal entspricht, zu jedem Zeitpunkt innerhalb der voreingestellten Dauerkonfiguriert ist; und
das Verarbeitungsmodul (62) speziell konfiguriert ist zum: Einholen, für den Eigenwertvektor zu jedem Zeitpunkt, eines Stresszustandswerts des Benutzers zu jedem Zeitpunkt basierend auf dem Eigenwertvektor und Bestimmen des kleinsten Stresszustandswerts des Benutzers innerhalb der voreingestellten Dauer basierend auf dem Stresszustandswert des Benutzers zu jedem Zeitpunkt innerhalb der voreingestellten Dauer; und
wobei der Eigenwertvektor eine Vielzahl von unterschiedlichen Eigenwertkomponenten umfasst und der Stresszustandswert des Benutzers zu jedem Zeitpunkt durch Durchführen einer gewichteten Summierung basierend auf jeder Eigenwertkomponente im Eigenwertvektor und einem jeder Eigenwertkomponente entsprechenden Gewichtungswert eingeholt wird.

5. Vorrichtung nach Anspruch 4, wobei das Einholungsmodul (61) außerdem zum Einholen von grundlegenden Informationen des Benutzers konfiguriert ist, bevor das Verarbeitungsmodul (12, 62) die Kalibrierungsinformationen basierend auf dem kleinsten Referenzspannungswert der Gruppe, zu der der Benutzer gehört, und dem kleinsten Stresszustandswert bestimmt; und
das Verarbeitungsmodul (12, 62) ferner konfiguriert ist zum: Bestimmen einer Gruppenkennung des Benutzers basierend auf den Basisinformationen und Abfragen einer Stresswertdatenbank basierend auf der Gruppenkennung des Benutzers, um den kleinsten Referenzbelastungswert der Gruppe, zu der der Benutzer gehört, zu bestimmen, wobei die Stresswertdatenbank eine Entsprechung zwischen einer Gruppenkennung und einem Referenzstressbereich speichert.

6. Vorrichtung nach einem der Ansprüche 4 oder 5, wobei das Kalibrierungsmodul (63) speziell konfiguriert ist zum: Einholen eines vorhergesagten Stresszustandswerts basierend auf dem Eigenwertvektor und Kalibrieren des vorhergesagten Stresszustandswerts unter Verwendung der Kalibrierungsinformationen, um den theoretischen Stresszustandswert des Benutzers einzuholen.

7. Computerlesbares Speichermedium, wobei das Computerspeichermedium Computeranweisungen speichert, die bei Ausführung auf einem Computer den Computer veranlassen, das Verfahren nach einem der Ansprüche 1 bis 3 durchzuführen.

## Revendications

1. Procédé d'évaluation et de calibrage du stress réalisé par un appareil d'évaluation et de calibrage du stress comprenant un module d'obtention (61), un module de traitement (12, 62) et un module de calibrage (13, 63), ledit procédé comprenant :
l'obtention (21), par le module d'obtention (61), d'un signal de paramètre physiologique d'un utilisateur ;
la détermination (22), par le module de traitement (12, 62), d'une plus petite valeur d'état de stress de l'utilisateur pendant une durée prédéfinie sur la base d'un vecteur de valeur propre du signal de paramètre physiologique ;
la détermination (23), par le module de traitement (12, 62), des informations de calibrage sur la base d'une plus petite valeur de stress de référence d'un groupe auquel appartient l'utilisateur et de la plus petite valeur d'état de stress ; et
le calibrage (24), par le module de calibrage (13, 63), à l'aide des informations de calibrage, d'une valeur d'état de stress, pour déterminer une valeur d'état de stress théorique de l'utilisateur ;
dans lequel la détermination d'une plus petite valeur d'état de stress de l'utilisateur pendant une durée prédéfinie sur la base d'un vecteur de valeur propre du signal de paramètre physiologique comprend :
l'obtention (31) d'un vecteur de valeur propre correspondant au signal de paramètre physiologique à chaque instant au cours de la durée prédéfinie ;
pour le vecteur de valeur propre à chaque instant, l'obtention (32) d'une valeur d'état de stress de l'utilisateur à chaque instant sur la base du vecteur de valeur propre ; et
la détermination (33) de la plus petite valeur d'état de stress de l'utilisateur pendant la durée prédéfinie sur la base de la valeur d'état de stress de l'utilisateur à chaque instant pendant la durée prédéfinie ; et
dans lequel le vecteur de valeur propre comprend une pluralité de composantes de valeur propre différentes, et la valeur d'état de stress de l'utilisateur à chaque instant est obtenue en réalisant une sommation pondérée sur la base de chaque composante de valeur propre dans le vecteur de valeur propre et d'une valeur de poids correspondant à chaque composante de valeur propre.

2. Procédé selon la revendication 1, dans lequel, avant la détermination des informations de calibrage basées sur une plus petite valeur de stress de référence d'un groupe auquel appartient l'utilisateur et la plus petite valeur d'état de stress, le procédé comprend également :
l'obtention (41) des informations de base sur l'utilisateur ;
la détermination (42) d'un identifiant de groupe de l'utilisateur sur la base des informations de base ; et
l'interrogation (42) d'une base de données de valeurs de stress sur la base de l'identifiant de groupe de l'utilisateur, pour déterminer la plus petite valeur de stress de référence du groupe auquel appartient l'utilisateur, dans lequel la base de données de valeurs de stress stocke une correspondance entre un identifiant de groupe et une plage de stress de référence.

3. Procédé selon l'une quelconque des revendications 1 à 2, dans lequel le calibrage, à l'aide des informations de calibrage, d'une valeur d'état de stress, pour déterminer une valeur d'état de stress théorique de l'utilisateur comprend :
l'obtention (51) d'une valeur d'état de stress prédite sur la base du vecteur de valeur propre ; et
le calibrage (52) de la valeur d'état de stress prédite à l'aide des informations de calibrage, pour obtenir la valeur d'état de stress théorique de l'utilisateur.

4. Appareil d'évaluation et de calibrage du stress, comprenant un module d'obtention (61), un module de traitement (12, 62) et un module de calibrage (13, 63), dans lequel le module d'obtention (61) est configuré pour obtenir (21) un signal de paramètre physiologique d'un utilisateur ;
le module de traitement (12, 62) est configuré pour :
déterminer (22) une plus petite valeur d'état de stress de l'utilisateur pendant une durée prédéfinie sur la base d'un vecteur de valeur propre du signal de paramètre physiologique, et déterminer (23) des informations de calibrage sur la base d'une plus petite valeur de stress de référence d'un groupe auquel appartient l'utilisateur et la plus petite valeur d'état de stress ; et
le module de calibrage (13, 63) est configuré pour calibrer (23), à l'aide des informations de calibrage, d'une valeur d'état de stress, pour déterminer une valeur d'état de stress théorique de l'utilisateur ;
dans lequel le module d'obtention (61) est en outre configuré pour obtenir un vecteur de valeur propre correspondant au signal de paramètre physiologique à chaque instant pendant la durée prédéfinie ; et
le module de traitement (62) est spécifiquement configuré pour : pour le vecteur de valeur propre à chaque instant, obtenir une valeur d'état de stress de l'utilisateur à chaque instant sur la base du vecteur de valeur propre, et déterminer la plus petite valeur d'état de stress de l'utilisateur pendant la durée prédéfinie sur la base de la valeur de l'état de stress de l'utilisateur à chaque instant pendant la durée prédéfinie ; et
dans lequel le vecteur de valeur propre comprend une pluralité de composantes de valeur propre différentes, et la valeur d'état de stress de l'utilisateur à chaque instant est obtenue en réalisant une sommation pondérée sur la base de chaque composante de valeur propre dans le vecteur de valeur propre et d'une valeur de poids correspondant à chaque composante de valeur propre.

5. Appareil selon une quelconque de la revendication 4, dans lequel le module d'obtention (61) est en outre configuré pour obtenir des informations de base sur l'utilisateur avant que le module de traitement (12, 62) ne détermine les informations de calibrage sur la base de la plus petite valeur de stress de référence du groupe auquel appartient l'utilisateur et la plus petite valeur d'état de stress ; et
le module de traitement (12, 62) est en outre configuré pour : déterminer un identifiant de groupe de l'utilisateur sur la base des informations de base, et interroger une base de données de valeurs de stress sur la base de l'identifiant de groupe de l'utilisateur, pour déterminer la plus petite valeur de stress de référence du groupe auquel l'utilisateur appartient, dans lequel la base de données de valeurs de stress stocke une correspondance entre un identifiant de groupe et une plage de stress de référence.

6. Appareil selon l'une quelconque de la revendication 4 ou 5, dans lequel le module de calibrage (63) est spécifiquement configuré pour : obtenir une valeur d'état de stress prédite sur la base du vecteur de valeur propre, et calibrer la valeur d'état de stress prédite à l'aide des informations de calibrage, pour obtenir la valeur théorique de l'état de stress de l'utilisateur.

7. Support de stockage lisible par ordinateur, dans lequel le support de stockage comprend des instructions qui, lorsqu'elles sont exécutées sur un ordinateur, amènent l'ordinateur à réaliser le procédé selon l'une quelconque des revendications 1 à 3.
